Europäisches Patentamt

(19) European Patent Office

Office européen des brevets

(11) Veröffentlichungsnummer: **0 012 926
B1**

(12) **EUROPÄISCHE PATENTSCHRIFT**

(45) Veröffentlichungstag der Patentschrift:
07.03.84

(21) Anmeldenummer: 79105091.7

(22) Anmeldetag: 11.12.79

(51) Int. Cl.³: **A 61 K 49/04**

(30) Priorität: 19.12.78 AT 9076/78

(43) Veröffentlichungstag der Anmeldung:
09.07.80 Patentblatt 80/14

(45) Bekanntmachung des Hinweises auf die Patenterteilung:
07.03.84 Patentblatt 84/10

(84) Benannte Vertragsstaaten:
AT BE CH DE FR GB IT LU NL SE

(56) Entgegenhaltungen:
CH - A - 273 245
CH - A - 373 866
CH - A - 375 841
DE - C - 919 908
FR - M - 3 658
FR - M - 5 986
FR - M - 6 777
GB - A - 834 517

(54) Röntgenkontrastmittellösungen.

(73) Patentinhaber: **Byk Gulden Lomberg Chemische Fabrik GmbH, Byk-Gulden-Strasse 2, D-7750 Konstanz (DE)**

(72) Erfinder: **Gottlob, Rainer, Prof. Dr., Kirchengasse 28, A-1070 Wien (AT)**

Die Erfindung betrifft intravasal einzuführende, verdickungsmittelfreie, wässrige, mit Blut mischbare Röntgenkontrastmittellösungen

Die Erfindung betrifft intravasal einzuführende, verdickungsmittelfreie, wässrige, mit Blut mischbare Röntgenkontrastmittellösungen.

Die Innenauskleidung der menschlichen und tierischen Blutgefässe (Endothel) stellt eine wichtige Struktur dar; bei ihrer Verletzung kann es zu Thrombenbildung kommen. Geringere Läsionen können zu Permeabilitätsstörungen und dadurch zu vermehrtem Austritt von Flüssigkeiten in die Gewebe führen.

Bei der röntgenologischen Darstellung von Blutgefässen werden wässrige Lösungen von iodhaltigen Verbindungen eingespritzt, um in den Gefässen eine erhöhte Absorption von Röntgenstrahlen zu erzeugen. Diese sogenannten Röntgenkontrastmittel sind hypertone Lösungen, die in der Regel deutlich nachweisbare Endothelschäden verursachen.

Durch Verminderung der hypertonen Eigenschaften der Kontrastmittellösungen, z.B. durch Verwendung nichtionischer Iodverbindungen wir Metrizamid oder durch die Verwendung von Iodverbindungen mit höherem Molekulargewicht wie Ioxaglinsäure, ist es gelungen, die Schädigungen am gesunden Endothel von normal durchbluteten Blutgefässen im Regelfall in vertretbaren Grenzen zu halten. Trotzdem können durch Kontrastmittel verursachte Zwischenfälle noch immer nicht mit Sicherheit ausgeschlossen werden. In besonderem Masse sind Endothelschädigungen beim Einbringen von Röntgenkontrastmittellösungen in Gefässe mit krankhaft verminderter bzw. behinderter Durchblutung, wie z.B. Varizen oder Verschlüssen der Beinvenen zu erwarten, da die Kontrastmittellösungen sich aufgrund der verminderten Durchströmung der Gefässe bedeutend länger in Kontakt mit dem Endothel befinden. Erschwerend kommt hierbei hinzu, dass das Endothel krankhaft veränderter Venen in der Regel vorgeschädigt und somit weniger belastbar ist, d.h. empfindlicher auf die Einwirkung des Röntgenkontrastmittels reagiert.

Die endothelschädigende Wirkung von Röntgenkontrastmitteln ist bekannt und kann nach der Methode von Gottlob und Zinner, Wien. Klin. Wschr. 77, 149 [1965] (Silberfärbung der überlebenden Rattenaorta nach Einwirken der Kontrastmittel, – Untersuchung der Aortenendothelien am aufgespannten Präparat von der Fläche her, – Methode der «en face-Präparate») semiquantitativ erfasst werden. Mit dieser Methode kann der Prozentsatz des geschädigten Areals der untersuchten Aorta angegeben werden.

Eine andere Form der durch Röntgenkontrastmittel verursachten Endothelschädigung lässt sich dadurch nachweisen, dass das Endothel nach dem Kontakt mit dem Kontrastmittel vermehrt mit Farbstoffen, wie Evans Blue oder Trypanblau angefärbt werden kann. Dies ist als Zeichen einer Permeabilitätsstörung zu werten (Gottlob, R. Amipaque-Workshop, 29. Mai 1978, Berlin, Excerpta Medica, Amsterdam–Oxford, 1978, S. 124–132).

Der Erfindung liegt nun die Aufgabe zugrunde, derartige intravasal einzuführende Röntgenkontrastmittel dahingehend zu verbessern, dass die Gefahr von Endothelschädigungen bedeutend herabgesetzt wird. Es wurde nun gefunden, dass die endothel- und permeabilitätsschädigenden Wirkungen derartiger intravasal einzuführender Röntgenkontrastmittel durch den Zusatz von körperverträglichen Tensiden stark vermindert werden kann. Der Zusatz von Tensiden zu Röntgenkontrastmitteln in Emulsions- oder Suspensionsform zur röntgenographischen Darstellung verschiedener Körperhöhlen ist bekannt. So wird in der AT-PS 218175 ein in Kapseln peroral zu applizierendes Gallenkontrastmittel beschrieben, dem zur besseren Löslichkeit ein Tensid zugesetzt wird. Aus der DE-OS 2216402 sind Öl-in-Wasser-Emulsionen zur röntgenographischen Untersuchung des Gastrointestinaltrakts bekannt, deren Ölphase aus iodhaltigen Verbindungen besteht, denen man emulsionsstabilisierende und schaumhemmende Tenside beifügt. In der CH-PS 373866 wird der Zusatz von nichtionischen Tensiden zu Röntgenkontrastmitteln in Form wässriger Suspensionen offenbart, wodurch eine grössere Haftfestigkeit dieser Suspensionen an der Oberfläche des Bronchialsystems oder des Uterus erreicht werden soll. In der Literaturstelle M. Guerbet, Thérapie, 1966, XXI, 1585–1592 wird eine ölige Röntgenkontrastmittelformulierung beschrieben, bei der durch Zusatz eines Tensids die Emulsionsbildung mit dem Blutserum verbessert werden soll, um die Gefahr von Embolien herabzusetzen. Aus der AT-PS 175974 sind wässrige Lösungen von iodierten Pyridonderivaten bekannt, die neben einem viskositätserhöhenden Zusatz noch ein Netzmittel zur Erhöhung der Haftfestigkeit an Körperhöhlen enthalten können. Der Zusatz von Tensiden zu wässrigen, mit Körperflüssigkeiten mischbaren intravasal zu verabreichenden Röntenkontrastmittellösungen ist somit neu und wird durch den Stand der Technik nicht nahegelegt.

Es wurde gefunden, dass es zweckmässig ist, körperverträgliche Tenside in solchen Mengen einzusetzen, dass die Grenzflächenspannung der erfindungsgemässen Röntgenkontrastmittellösungen gegenüber den Gefässwänden ungefähr auf die Grenzflächenspannung herabgesetzt wird, die zwischen Blut und den Gefässwänden herrscht.

Gegenstand der Erfindung sind daher intravasal einzuführende, verdickungsmittelfreie, wässrige, mit Blut mischbare Röntgenkontrastmittellösungen, die durch den Zusatzt von pharmakologisch unbedenklichen Tensiden in einer solchen Menge, dass die Grenzflächenspannung gegenüber den Körpergefässwänden ungefähr auf die Grenzflächenspannung herabgesetzt wird, die zwischen Blut und den Gefässwänden herrscht, gekennzeichnet sind.

Unter intravasal einzuführenden Röntgenkontrastmittellösungen sind solche für die Arteriographie und die Phlebographie, zu verstehen.

Weiterhin wurde gefunden, dass bei osmotisch hypertonen Röntgenkontrastmitteln zweckmässigerweise die Grenzflächenspannung auf Werte herabgesetzt wird, die unter denen des Blutes gegenüber den Gefässwänden liegen. Durch diese erfindungsgemässe Massnahme wird gleichzeitig die auf osmotische Schrumpfung zurückzuführende Endothelschädigung vermindert.

Einige Röntgenkontrastmittel, die nicht ionisch dissoziieren und daher einen geringeren (aber im Vergleich zum Blut immer noch erhöhten) osmotischen Druck aufweisen, wie z.B. Metrizamide (2-(3-Acetamido-5N-methylacetamide-2,4,6-triiodobenzamido)-2-deoxy-D-glucose) bewirken, möglicherweise infolge einer gewissen Lipophilie, eine durch erhöhte Anfärbbarkeit nachweisbare Schädigung der Gefässwand. Hier kann durch Auswahl besonders hydrophiler Tenside nicht nur die Grenzflächenspannung der des Blutes angeglichen werden, sondern zugleich auch die erhöhte Lipophilie, die anscheinend zu Permeabilitätsstörungen führt, herabgesetzt werden.

Unter körperverträglichen Tensiden sollen diejenigen Tenside verstanden werden, welche bei der Einstellung der erforderlichen Grenzflächenspannung weder hämolytische noch toxische Reaktionen zeigen. Die erforderliche Konzentration hängt weitgehend von der Wahl des Tensids ab, wobei von einem durch Zusatz von Röntgenkontrastmitteln gegebenen überhöhten Wert für die Grenzflächenspannung ausgegangen werden muss. Auch die Erhöhung der Grenzflächenspannung durch Röntgenkontrastmittel ist für die einzelnen diagnostisch gebräuchlichen Kontrastmittel verschieden. Allen Röntenkontrastmitteln ist jedoch eine hypertonische Reaktion gemeinsam, welche durch die Herabsetzung über die Grenzflächenspannungswerte des Blutes hinaus zumindest teilweise kompensiert werden kann.

Die erfindungsgemässe Einstellung der Grenzflächenspannung kann durch geeignete Mischung von Röntgenkontrastmitteln unterstützt werden, so dass die für die Herabsetzung der Grenzflächenspannung auf den gewünschten Wert erforderliche Menge an Tensiden minimiert werden kann.

Die Grenzflächenspannung von herkömmlichen Röntgenkontrastmittellösungen gegenüber der Gefässwand liegt in der Regel zwischen 18 und $28.10^{-5}$ N/cm. Eine gute Endothelverträglichkeit wird erfindungsgemäss insbesondere durch eine Absenkung dieser Grenzflächenspannung durch Zugabe von Tensiden auf Werte unter $16.10^{-5}$ N/cm erreicht. Die entsprechenden Grenzflächenspannungswerte für Blut liegen in der Grössenordnung von 8 bis $15.10^{-5}$ N/cm und zumeist etwa bei $12.10^{-}$ N/cm. Da die Bestimmung der Grenzflächenspannung gegenüber der Gefässwand nicht ohne weiteres möglich ist, wurde gefunden, dass eine Referenzsubstanz aus 0,5 vol% Ölsäure in reinem Olivenöl in seiner Grenzflächenspannung gegen Blutflüssigkeit etwa der Gefässwand entspricht, und dass daher gegenüber dieser Referenzsubstanz gemessene Werte im wesentlichen den angenommenen Werten gegenüber der Gefässwand entsprechen. Im Sinne der vorliegenden Erfindung sind daher unter Grenzflächenspannungen zwischen Röntgenkontrastmitteln bzw. Körperflüssigkeiten und Gefässwänden solche zu verstehen, die gegen eine Lösung von 0,5 Vol% Ölsäure in reinem Olivenöl gefunden werden. Die Messung der Grenzflächenspannung kann nach den üblichen Methoden erfolgen, z.B. stalagmometrisch nach Harkins und Brown.

Als Tenside kommen anionische, kationische und nichtionische Tenside in Frage, wobei nichtionische Tenside bevorzugt sind. Ionische Tenside haben naturgemäss eine ausgeprägtere grenzflächenspannungssenkende Wirkung als nichtionische Tenside, sie führen jedoch bereits in wesentlich geringeren Konzentrationen zu Hämolyse. In jedem Fall empfiehlt es sich, die Hypertonie der Tenside durch geeignete Überkompensation, dass heisst Herabsetzung unter die physiologischen Werte des Blutes für die Grenzflächenspannung, zu berücksichtigen.

Erfindungsgemäss wird die Grenzflächenspannung gegenüber der Gefässwand zumindest auf $16.10^{-5}$ N/cm durch die Tenside herabgesetzt. Besonders günstige Ergebnisse werden bei Werten im Bereich von 8 bis $15.10^{-5}$ N/cm, insbesondere bei Werten von etwa $12.10^{-5}$ N/cm, erzielt.

Ein wesentliches Kriterium für die Brauchbarkeit eines Tensids ist durch die sogenannte HLB-Zahl gegeben. Diese Zahl stellt die «Hydrophilie-lipophilie-balance» dar und ein hoher Zahlenwert entspricht einer ausgeprägten Hydrophilie, während ein niederer Zahlenwert für ausgeprägte Lipophilie steht. Vorzugsweise wird durch Zusatz geeigneter hydrophiler Tenside die Lipophilie auf eine HLB-Zahl von grösser als 15, insbesondere 25 bis 35, vorzugsweise etwa 29, herabgesetzt, wofür vor allem Blockmischpolykondensate aus Propylenglykol und Polyethylenglykol sich als geeignet erwiesen haben.

Als Tenside sind vor allen Dingen die biologisch gut verträglichen Lecithine mit mehrfach ungesättigten Fettsäuren geeignet. Bei solchen Tensiden kann eine relativ hohe Konzentration, insbesondere eine Konzentration von etwa 0,1 bis 1 g/100 ml, vorzugsweise etwa 0,5 g/100 ml, eingestellt werden.

Ein weiteres geeignetes Tensid ist Polyoxyethylen-sorbitan-monooleat. Die erforderliche Menge richtet sich hierbei jeweils nach dem gewählten Tensid, ist aber auch vom gewählten Röntgenkontrastmittel beeinflusst. Übliche Röntgenkontrastmittel für die Angiographie sind substituierte Triiodbenzoesäuren, wobei im speziellen beispielsweise das Methylglucaminsalz der Diacetyl-3,5-diamino-2,4,6-triiodbenzoesäure, das Monoethanolamin-, Methylglucamin- bzw. Natriumsalz der 5-Acetamido-N-(2-hydroxyethyl)-2,4,6-triiodsophthalamsäure genannt werden sollen.

Erfindungsgemäss bevorzugt verwendete Tenside sind die nichtionogenen, wie z.B. natürliche Lecithine, Polyethoxysorbitanfettsäureester (im Handel z.B. unter dem Warenzeichen Tween®), Polyethoxy-Ricinusfettsäureglycerinester (im

Handel z.B. unter dem Warenzeichen Cremophor EL, RH 40 und RH 60®) und Polyoxyethylenpolyoxypropylen-Polymere (im Handel z.B. unter dem Warenzeichen Pluronics®).

Die Erfindung wird nachfolgend an Hand von Ausführungsbeispielen näher erläutert.

Beispiel

Ampullen (30 ml) enthaltend 19,8 g Megluminloxitalamat.

Zusammensetzung

300 Liter Lösung enthalten:

| | |
|---|---|
| Ioxitalaminsäure | 151,98 kg |
| Methylglucamin | 46,02 kg |
| EPL US | 1,50 kg |
| Natriumdisulfit | 0,03 kg |

Wasser zur Injektion auf 300 l (ca. 100 l)

In 100 l Wasser zur Injektion von 80°C werden mittels eines schnelllaufenden Rührers das EPL US gelöst und anschliessend die Ioxitalaminsäure suspendiert. Nachdem das Methylglucamin langsam zugesetzt wurde, wird die klare Lösung auf ca. 40°C abgekühlt und das Natriumdisulfit zugegeben. Durch Zugabe von Ioxitalamin oder Methylglucamin wird ein pH Wert von 7,0 ± 0,5 eingestellt. Die Lösung wird sterilfiltriert und in 30 ml Ampullen gefüllt. Es wird bei 120°C 20 Minuten sterilisiert.

Es wurden eine Reihe von anionischen, kationischen und nichtionischen Detergenzien getestet, wobei die Dosierung so erfolgte, dass Hämolyse nicht beobachtet werden konnte. Signifikante Unterschiede gegenüber Röntgenkontrastmittel ohne Zusatz an grenzflächenaktiven Stoffen wurden bei Zugabe von Lecithin mit mehrfach ungesättigten Fettsäuren bzw. durch Ultraschall suspendierten essentiellen Phospholipiden (EPL US) beobachtet, wenn die Endkonzentration etwa 0,1 bis 1 g/100 ml, vorzugsweise etwa 0,5 g/100 ml, dieser Zusätze beträgt. Bei Zugabe von 75 mg/100 ml Polyoxyethylen-sorbitan-monooleat zu der Lösung des Methylglucaminsalzes der Diacetyl-3,5-diamino-2,4,6-triiodbenzoesäure wurde ebenso wie bei Zusatz von etwa 50 mg/100 ml eines Octylphenol-polyethylenglycoletherformaldehyd-polymer, oder 0,4 mg/100 ml Cetylpyridinchlorid, oder 3 mg/100 ml Cetylpyridinchlorid, oder 3 mg/100 ml Natriumdodecylsulfat, eine signifikante Herabsetzung der Grenzflächenspannung gegenüber der Gefässwand auf Werte unterhalb 16.10⁻⁵ N/cm beobachtet. Keines dieser Tenside zeigte irgendwelche nachteilige Effekte nach der Verabreichung.

Gegenstand der Erfindung ist auch die Verwendung von pharmakologisch unbedenklichen Tensiden als Zusatz zu intravasal einzuführenden, verdickungsmittelfreien, wässrigen, mit Blut mischbaren Röntgenkontrastmittellösungen.

**Patentansprüche**

1. Intravasal einzuführende verdickungsmittelfreie, wässrige, mit Blut mischbare Röntgenkontrastmittellösungen, gekennzeichnet durch einen Zusatz von pharmakologisch unbedenklichen Tensiden in einer solchen Menge, dass die Grenzflächenspannung gegenüber den Körpergefässwänden ungefähr auf die Grenzflächenspannung herabgesetzt wird, die zwischen Blut und den Körpergefässwänden herrscht.

2. Röntgenkontrastmittellösungen nach Anspruch 1, gekennzeichnet durch eine Grenzflächenspannung gegenüber den Körpergefässwänden von maximal 16.10⁻⁵ N/cm, vorzugsweise von maximal 12.10⁻⁵ N/cm.

3. Röntgenkontrastmittellösungen nach Anspruch 1, gekennzeichnet durch eine Grenzflächenspannung gegenüber der Gefässwand von 8 bis 15.10⁻⁵ N/cm, vorzugsweise von etwa 12.10⁻⁵ N/cm.

4. Röntgenkontrastmittellösungen nach einem der Ansprüche 1 bis 3, dadurch gekennzeichnet, dass als Tensid ein Lecithin, insbesondere ein Lecithin mit mehrfach ungesättigten Fettsäuren in einer Menge von 0,1 bis 1 g/100 ml, vorzugsweise etwa 0,5 g/100 ml, enthalten ist.

5. Röntenkontrastmittellösungen nach einem der Ansprüche 1 bis 3, dadurch gekennzeichnet, dass als Tensid Polyoxyethylen-sorbitan-fettsäureester, insbesondere Polyoxyehtylen-sorbitan-monooleat, vorzugsweise in einer Menge von 75 mg/100 ml enthalten ist.

6. Röntgenkontrastmittellösungen nach einem der Ansprüche 1 bis 3, dadurch gekennzeichnet, dass als Tensid ein Block-mischpolykondensat aus Propylenglykol und Polyethylenglykol im Verhältnis 2:9 enthalten ist.

7. Röntgenkontrastmittellösungen nach einem der Ansprüche 1 bis 3, dadurch gekennzeichnet, dass als Tensid ein Octylphenol-polyethylenglycoletherformaldehyd-polymer, vorzugsweise in einer Konzentration von 50 mg/100 ml enthalten ist.

8. Röntgenkontrastmittellösungen nach einem der Ansprüche 1 bis 3, dadurch gekennzeichnet, dass als Tensid Cetylpyridinchlorid in einer Menge von vorzugsweise 0,4 mg/100 ml enthalten ist.

9. Röntgenkontrastmittellösungen nach einem der Ansprüche 1 bis 4, dadurch gekennzeichnet, dass als Tensid essentielle Phospholipide, vorzugsweise durch Ultraschall fein dispergiert, in einer Menge von vorzugsweise 0,05 bis 0,5 g/100 ml enthalten sind.

10. Röntenkontrastmittellösungen nach einem der Ansprüche 1 bis 9, dadurch gekennzeichnet durch eine HLB-Zahl von grösser als 15, vorzugsweise etwa 29, für dessen Einstellung vorzugsweise ein hydrophiles Tensid zugesetzt ist.

11. Röntgenkontrastmittellösungen nach einem der Ansprüche 1 bis 10, dadurch gekennzeichnet, dass sie als Kontrastmittel 5-Acetamido-N-(2-hydroxyethyl)-2,4,6-triiodisophthalamsäure oder ihre Salze enthalten.

12. Verwendung von pharmakologisch unbedenklichen Tensiden als Zusatz zu verdickungsmittelfreien, wässrigen, mit Blut mischbaren intravasal zu verabreichenden Röntgenkontrastmittellösungen.

## Claims

1. Aqueous X-ray contrast media solutions which are to be introduced intravasally and which are miscible with blood and which are free of viscosity increasing agents, characterised by an addition of pharmacologically acceptable surface-active agents in an amount such that the interfacial tension with respect to the walls of the vessels in the body is approximately reduced to the interfacial tension which prevails between the blood and the vessel walls of the body.

2. X-ray contrast media solutions according to Claim 1, characterised by an interfacial tension with respect to the vessel walls of the body of at most $16.10^{-5}$ N/cm, preferably of at most $12.10^{-5}$ N/cm.

3. X-ray contrast media solutions according to Claim 2, characterised by an interfacial tension with respect to the vessel wall of 8 to $15.10^{-5}$ N/cm, preferable of about $12.10^{-5}$ N/cm.

4. X-ray contrast media solutions according to one of Claims 1 to 3, characterised in that they contain a lecithin, in particular a lecithin with polyunsaturated fatty acids, in an amount of preferably about 0.5 g/100 ml as the surface-active agent.

5. X-ray contrast media solutions according to one of Claims 1 to 3, characterised in that they contain polyoxyethylene sorbitane fatty acid esters, in particular polyoxyethylene sorbitane monooleate, preferably in an amount of 75 mg/100 ml, as the surface-active agent.

6. X-ray contrast media solutions according to one of Claims 1 to 3, characterised in that they contain a block copolycondensate of propylene glycol and polyethylene glycole, in the ratio 2 : 9, as the surface-active agents.

7. X-ray contrast media solutions according to one of Claims 1 to 3, characterised in that they contain an octylphenyl-polyethylene glycol ether-formaldehyde polymer, preferably in a concentration of 50 mg/100 ml, as the surface-active agent.

8. X-ray contrast media solutions according to one of Claims 1 to 3, characterised in that they contain cetylpyridine chloride in an amount of preferably 0.4 mg/100 ml as the surface-active agent.

9. X-ray contrast media solutions according to one of Claims 1 to 4, characterised in that they contain essential phospholipids, preferably finely dispersed by ultrasound, in an amount of preferably 0.05 to 0.5 g/100 ml as the surface-active agent.

10. X-ray contrast media solutions according to one of Claims 1 to 9, characterised by an HLB number of greater than 15, preferably about 29, for the establishment of which a hydrophilic surface-active agent is preferably added.

11. X-ray contrast media solutions according to one of Claims 1 to 10, characterised in that they contain as contrast medium 5-acetamid-N-(2-hydroxyethyl)-2,4,6-triiodoisophthalamic acid or its salts.

12. Use of pharmacologically acceptable surface-active agents as an additive to aqueous X-ray contrast media solutions which are to be introduced intravasally and which are miscible with blood and which are free of viscosity increasing agents.

## Revendications

1. Solution aqueuse d'agent de contraste aux rayons X pour l'introduction par voie intravasculaire dépourvue de produits d'épaississement et miscible au sang, caractérisée en ce qu'elle contient un dérivé tensio-actif pharmacologiquement compatible en quantité telle que sa tension superficielle vis-à-vis de la paroi corporelle est abaissée au niveau de celle qui règne entre le sang et les parois corporelles.

2. Solution d'agent de contraste selon la revendication 1, caractérisée en ce que sa tension superficielle maximale vis-à-vis de parois corporelles est de $15.10^{-5}$ N/cm et préférentiellement de $12.10^{-5}$ N/cm.

3. Solution d'agent de contraste selon la revendication 1, caractérisée en ce que sa tension superficielle vis-à-vis de parois corporelles est comprise entre 8 et $15.10^{-5}$ N/cm et de préférence aux environs de $12.10^{-5}$ N/cm.

4. Solution d'agent de contraste selon l'une quelconque des revendications 1 à 3, caractérisée en ce qu'elle contient comme tensio-actif de la lécithine et plus particulièrement de la lécithine contenant des acides gras polyinsaturés, la quantité dudit tensio-actif étant comprise entre 0,1 et 1 g/100 ml, et de préférence aux environs de 0,5 g/100 ml.

5. Solution d'agent de contraste selon l'une quelconque des revendications 1 à 3, caractérisée en ce qu'elle contient comme tensio-actif un ester d'acide gras de polyoxyéthylène-sorbitol et plus particulièrement du monooléate de polyoxyéthylène-sorbitol, avantageusement environ 75 mg/100 ml.

6. Solution d'agent de contraste selon l'une quelconque des revendications 1 à 3, caractérisée en ce qu'elle contient comme tensio-actif un copolymérisat en masse de propylène-glycol et de polyéthylène-glycol dans la proportion de 2 à 9.

7. Solution d'agent de contraste selon l'une quelconque des revendications 1 à 3, caractérisée en ce qu'elle contient comme tensio-actif un polymère d'octylphénol-étherpolyéthylèneglycolique-formaldéhyde, la concentration dudit tensio-actif étant de préférence aux environs de 50 mg/100 ml.

8. Solution d'agent de contraste selon l'une quelconque des revendications 1 à 3, caractérisée en ce qu'elle contient comme tensio-actif le chlorure de cétylpyridine, avantageusement 0,4 mg/100 ml.

9. Solution d'agent de contraste selon l'une quelconque des revendications 1 à 4, caractérisée en ce qu'elle contient comme tensio-actif des phospholipides essentiels avantageusement dispersés à l'aide d'ultra-sons et en une quantité avantageusement comprise entre 0,05 et 0,5 g/100 ml.

10. Solution d'agent de contraste selon l'une quelconque des revendications 1 à 9, caractérisée en ce que sa valeur HLB est supérieure de 15 et de préférence supérieure à 29 et en ce qu'on utilise avantageusement pour régler ladite valeur un tensio-actif hydrophile.

11. Solution d'agent de contraste selon l'une quelconque des revendications 1 à 10, caractérisée en ce qu'elle contient en tant qu'agent de contraste l'acide 5-acétamido-N-(2-hydroxyéthyl)-2,4,6-triiodoisophtalique ou un de ses sels.

12. Utilisation des dérivés tensio-actifs pharmacologiquement compatibles en tant qu'additif aux solutions aqueuses d'agent de contraste aux rayons X dépourvues de produits d'épaississement et destinées à l'introduction par voie intravasculaire.